# EUROPEAN PATENT APPLICATION

(11) **EP 1 992 946 A1**
(43) Date of publication of application: **19.11.2008**
(21) Application number: 07425290.9
(22) Date of filing: 18.05.2007
(51) Int. Cl.: G01N 33/18, G01N 1/10

(54) **Automated sampler device to carry out analytical experiments, particularly in waste water treatment plants**

(71) Applicant: Spes Società Cooperativa per azioni, 60044 Fabriano (AN) (IT); Politecnico Di Milano, 20133 Milano (IT)
(72) Inventor: Canziani, Roberto c/o Politecnico di Milano, 20133 Milano (IT); Ficara, Elena c/o Politecnico di Milano, 20133 Milano (IT); Esposti, Francesco c/o Spes Soc. Co. per azioni, 60044 Fabriano (Ancona) (IT); Fiocchi, Nicola c/o Spes Soc. Co. per azioni, 60044 Fabriano (Ancona) (IT); Lancioni, Mirco c/o Spes Soc. Co. per azioni, 60044 Fabriano (Ancona) (IT); Mariani, Simone c/o Spes Soc. Co. per azioni, 60044 Fabriano (Ancona) (IT); Pirani, Massimiliano c/o Spes Soc. Co. per azioni, 60044 Fabriano (Ancona) (IT); Quintaba, Andrea c/o Spes Soc. Co. per azioni, 60044 Fabriano (Ancona) (IT); Ratini, Paolo c/o Spes Soc. Co. per azioni, 60044 Fabriano (Ancona) (IT); Ciappelloni, F. c/o Spes Soc.Coop. per azioni, 60044 Fabriano (Ancona) (IT)
(74) Representative: Gallarotti, Franco

(57) **Abstract**

Automatic sampler device for an activated sludge waste water purification plant, comprising:
- an electronic control system (15),
- fitting means (IN1, IN2) for the hydraulic connection of the device to a purification plant (1), for the purposes of a controlled taking, from this latter, of an influent sample, or a first analyte, and a sludge sample, or a second analyte,
- storage means (70) for one or more reagents and/or titrants,
- a chemical reactor (50), in a fluid communication with said fitting means (IN1, IN2) and with said storage means (70),
- actuator means, which can be controlled by said control means (15) for the purpose of introducing and/or conditioning within said chemical reactor (50) at least a dose of a said analyte and at least a dose of reagent or titrant,
- sensor means, operatively associated with said chemical reactor (101), for the detection of quantities relative to the content thereof, said sensor means being used by the control system (15) in the ambit of at least a control action on said actuator means.

The control system (15) is arranged for implementing within said chemical reactor (110), through said sensor means and said actuator means, one or more analytical experiments in an automated way, using at least a set-point titration methodology selected from pH-Stat titration and DO-Stat titration.

## Description

### Field of the invention

The present invention relates to the field of monitoring and control of plants and processes for the purification of industrial and/or civil type-waste water and it relates, particularly, to the on-line and real-time monitoring of such plants and processes.

### Definitions

***Continuous purification plants*** are those distinguished by a load of the entering sewage and by a discharge of the exiting purified effluent occurring in a continuous way over time, with a hydraulic flow rate which varies depending on the sewer load of the system served by the purifier, the daily and seasonal variations and the rain events. The plant engineering dimensions, in particular of the oxidation and sedimentation tanks, are proportioned with the entering load and are designed over-dimensioned according to proper safety factors.

***Sequencing purification plants*** (SBR: *Sequencing Batch Reactor)* are batch plants in which the sewage load and the discharge of the purified effluent occur in an intermittent way during the space of a day. The whole purification process takes place in a same tank, through following steps which occur over time in a sequential way. They are purifiers characterized by a remarkable working flexibility and effectiveness in treating waste water with flow rates and compositions sometimes extremely varying, as well as very interesting because of the reduced plant engineering encumbrance (thanks to the absence of sedimentators). This allows to carry out economical and effective plants, also where the available areas are limited.

***Anaerobic digestion plants*** are continuous or batch-type plants in which a purification biological process takes place in conditions lacking of oxygen (anaerobiosis). The process, mainly suggested when sullages very rich in organic substance have to be treated, is able to carry out, in parallel with the biological purification, heat and biogas production, mainly consisting of a methane and carbon dioxide mixture. Such plant engineering typology finds a wide employ in the treatment of zootechnical and food industry sullages.

By the term ***biomass*** (microorganisms consortium) is understood to mean the solid fraction existing in the tanks of the biological purification plant, consisting of complex bacterial colonies and multicellular microorganisms mixtures which develop and specialize within each plant. Such mixtures can aggregate in structures easily separable from the liquid phase by sedimentation or filtration, in the common slang called "flakes", or in a bacterial film adherent to an inert support made of a stone material or synthetic resins, in the slang called "biofilm", depending on the type of biological process. The remaining part of the solid fraction consists of thin particles which have overcome the primary filtration process, which can be inert or biodegradable. The complex of biomass and thin particles constitutes the ***sludge*** of the plant.

Plants and purification processes of waste water of a biological type can be:
- at ***suspended biomass,*** also called ***in activated sludge*** (ASP: *Activated Sludge Process),* both with a continuous flow (CF-ASP: *Continuous Flow - Activated Sludge Process)* and with a batch flow (SBR-ASP: *Sequencing Batch Reactor - Activated Sludge Process),* independently of the separation mode between liquid phase and sludges (for example: flotation, sedimentation or membrane);
- at ***adhered biomass,*** of a fixed-bed type (bio-filters), of a moving-bed type (MBBR: *Moving Bed Biofilm Reactor)* and/or of a fluidized-bed type (FBR: *Fluidized Bed Reactor).*

By the term ***kinetic parameters*** is understood to mean each quantity describing the evolution over time of a chemical and/or biochemical reaction.

By the term ***volatile fatty acids*** (VFA) short-chained acids of an organic origin are designed, formed by hydrolysis of organic substrates.

By the term ***titrants*** are understood to mean reagent solutions dosed in order to maintain at the set-point the chemical parameter used for the measurement or the estimation of the measured quantity.

By the term ***reagents*** are understood to mean all the other solutions required for the test development, dosed in moderate quantities at determined times based on the selected and configured method.

By the term ***analyte*** is understood to mean each of the samples collected in an exact point of a given plant for carrying out some tests.

By the term ***influent*** is understood to mean the sewage entering the purification plant.

By the term ***effluent*** is understood to mean the purified liquid exiting from the purification plant.

By the term ***activity*,** referred to a bacterial and/or cell population, the removal rate exerted by said population towards at least a substrate is designated, generally expressed as equivalent concentration of a removed substrate in the time unit.

By the term ***respirometry*** a determination method of the aerobic biomasses activity is designated, through the observation of the oxygen profile dissolved in the system under examination in which periodical aeration cycles in controlled conditions are applied (OUR: *Oxygen Uptake Rate). A **respirometry experiment*** allows the monitoring of the kinetics of an oxidation process generally developed by the action of bacterial populations.

### State of the art

So far, biotechnological applications are not so much applied in the monitoring and control field of water treatment processes.

One of the more diffuse processes in the ambit of the waste water biological purification, both of a civil and industrial origin, is the one called with *activated sludges* and is mainly based on a (continuous or batch) process wherein organic compounds are removed from the liquid phase through the action of suspended biomasses, which are hold in the system by separation, generally carried out with gravity in a sedimentator.

The considerable qualitative variations of the influent entering the plant, due to the discontinuity of the civil drainages and the processing which produce industrial sewages, or the introduction to the plant of toxic or overload due to extremely concentrated sewages, can sensibly compromise the effectiveness of the biological treatments. The immediate effect in such situations is the raising of pollutant concentrations in the purified effluent and the non-conformity with the limits permitted to the outlet.

In modem biological purification plants, the control of the kinetic parameters on the activated biomass can be carried out with different methods, also as a function of the kind of plant. Said kinetic parameters characterize the biochemical processes which involve the bacterial colonies existing in the biomass. The final effect of said processes is the degradation of the biodegradable substances existing in the sewage in simpler and non polluting compounds, such as for example the gaseous nitrogen.

The main kinetic parameters useful for the purification process control are:
- the measure of the activity of a single species, with this meaning the specific rate with which a substrate is transformed by the biomass in an end product;
- the growth and decay constants of the single species constituting the biomass;
- the cell yield coefficients, corresponding with the formed biomass per unit of removed substrate.

In plants of large dimensions, the presence of a laboratory through which a series of analyses are periodically carried out which, only in some cases, include also specific tests for the control of the kinetic parameters is generally foreseen. These tests are carried out by using respirometric techniques, or by monitoring over time the evolution of the concentrations of metabolites and substrates involved in the biological reactions entrusted with the degradation of the polluting substances.

In plants with middle or small-middle dimensions, in which an analyses laboratory is normally not present, the control of the kinetic parameters have to be limited to the analysis of instant samples collected by operators or, at best, through automatic samplers which collect average samples during 24 or 48 hours, stored in an air-conditioned environment, for then being analyzed in the laboratory.

In plants with small dimensions, kinetic parameters are generally not monitored and the analyses carried out are restricted to the periodical controls provided for by law on the exiting effluent.

The continuously working CF-ASP plants are the most widespread, even if those of the SBR-ASP type (sequential) present higher features in terms of effectiveness and flexibility with the change of the working conditions. De facto, the thing that has limited very much the diffusion of this second kind of plant, so far, has been the kind of required control. In fact, for the purposes of the process optimization, SBR plants require a more accurate control of the kinetic parameters in order to allow the management of the different work time intervals ascribed to the different sequential steps. An alternative and compromise approach commonly used is the one of setting fixed and cautiously wide time intervals for the different steps, however reducing the maximum treatable flow rates and the quality of the exiting sewage, accordingly increasing the energy consumption.

Another criticality of SBR plants is that, if required precautions are not adopted, they can result more vulnerable in the presence of inhibiting or toxic substances in the sewage to be treated, because of the missed dilution effect caused by the batch mode of the load.

The prevailing approach for the control of continuous or batch purification plants is based on continuous acquisition systems of working data, such as hydraulic flow rates, temperature, pH, oxide-reduction potential, dissolved oxygen, ammonia, nitrates. The main kinds of followed approaches are the following:
- real-time data acquisition systems, which manage a complex of sensors applied to the plant and intended for the measurement of chemical-physical parameters; the estimation of kinetic parameters to be monitored is obtained through following complex indirect processing of the acquired parameters;
- automatic sampling systems, which collect samples on which respirometric tests, spectro-photometric analyses or analyses based on the use of ion-selective sensors are subsequently carried out, which, by acquiring data related to the biomass activity, remarkably simplify the complexity of the interpretative patterns of the biological processes.

In case of real-time data acquisition systems relating to the first type of approach mentioned, the commonly used methods for the estimation of the kinetic parameters can be sometimes inadequate, as they rely on the measurement of the concentrations of the chemical species involved in the process. From the single concentrations, in fact, it is often difficult to go back to kinetic data ascribable to specific bacterial populations, as it often happens that a certain chemical species is involved in more contemporaneous reactions, not depending only from the activity of the searched population. In these cases, the final result is almost never reliable, not even by taking proper precautions.

The fact that a continuous plant operates in a stationary state mitigates this problem, even if there is always a high probability that an external cause modifies the parameters under control, invalidating the measurements. To this, the uncertainty of the representativeness of the point measurement, with respect of the effective distribution in the space and time of the measured variables has to be added.

In case of automatic sampling systems, concerning the second approach above mentioned, the measurement of the kinetic parameters can be obtained with different methods. One of the most rigorous method provides for the use of automated respirometric techniques (on-line respirometers), based on the measurement of the bacterial respiration rate.

Respirometers, however, have the defect of being unable to discriminate between autotroph activity (responsible of the oxidation of the reduced nitrogenous forms) and heterotroph (responsible of the oxidation of the organic compounds and the reduction of nitrates to molecular nitrogen).

Concerning this, it has to be pointed out that, in a biological purification process, the nitrogen removal usually takes place through an oxidation phase of the ammonia nitrogen to nitrate (nitrification), wherein two autotroph bacterial populations are involved, and a following denitrification phase, in which nitrates are reduced from heterotroph bacteria to molecular nitrogen, liberated in the atmosphere.

The activity of the nitrifying bacteria is much more sensitive to the temperature action and inhibiting agents also in a small concentration, than the one of heterotroph bacteria which oxidize the organic substance expressed as COD (*Chemical Oxygen Demand*). In comparison with these latter, moreover, nitrifying bacteria are distinguished by much slower growth kinetics, as well as by a small cell yield per unit of removed substrate. A loss of concentration of such biomass, not timely detected, can lead to the interruption of the removal process.

At present, the activity of these specific bacteria is estimated through the monitoring of the involved nitrogenous chemical species, with the aid of ion-selective sensors and spectro-photometric probes. However, these two classes of sensors have both the defect of providing the kinetic data only by an indirect way and, furthermore, they are distinguished by a poor robustness and require a complex maintenance due to the need of filtering the analyte which is collected for the analysis.

### Summary of the invention

The present invention has the aim of solving one or more of the drawbacks mentioned above.

In this frame, an aim of the present invention is to indicate a sampler device which allows to carry out, in an automated way, precise parameter detections in waste water purification plants, such as bacterial activity, effectiveness of nitrogenous compounds removal, inhibition and concentration events of the pollutants to be treated (COD, NH₄, NOₓ, etcetera).

Another aim of the invention is to indicate a sampler device which allows to automatically carry out the monitoring of the bacterial activity and the measurement of the quality of waste water entering a purification plant, to timely detect events which could compromise the effectiveness of the biological processes and, accordingly, carry out corrective measures (for example: dosage of reagents, such as activated carbon, or diversion of waters entering a temporary accumulation basin), before the biological process is compromised.

Another aim of the invention is to indicate an automatic sampler device capable of discriminating the contributions between autotroph and heterotroph activity, and therefore accurately control the whole removal process of the nitrogen. For SBR plants this is a fundamental aspect, all the purification process phases having to occur in a single tank and SBR plants being designed for managing process conditions very different therebetween.

Another aim of the invention is to indicate an automatic sampler device which allows, when used for the purposes of a purification plant management, to obtain a high purification effectiveness, a reduction of the maintenance costs and a continuous monitoring on the biomasses, so as to prevent malfunction events which would compromise the purification process and therefore the respect of the parameters on the purified effluent.

Another aim of the invention is to indicate such an automatic sampler device being of limited cost and featuring a high flexibility of use.

These and other aims, which will result more apparent below are obtained, according to the invention, by a sampler device having the features of the appended claims. The claims form an integral part of the technical teaching provided herein concerning the invention.

### Brief description of the drawings

Features and advantages of the invention will result apparent from the following description, carried out with reference to the enclosed drawings, given by mere way of non limiting example, in which:
- figure 1 is a diagrammatic representation of a purification plant associated with a sampler device according to the invention;
- figure 2 is a diagrammatic representation of a possible complex of program modules of the sampler device according to the invention;
- figure 3 is a partial and diagrammatic front view of a sampler device according to the invention;
- figure 4 is a partial and diagrammatic section of the sampler device of figure 3;
- figure 5 is a diagrammatic view of an analysis reactor of the device of figure 3;
- figure 6 is a diagrammatic view of a sampling vessel of the device of figure 3;
- figure 7 is a diagrammatic representation of part of a dosage system of the sampler device of figure 3.

### Description of examples of preferred embodiments

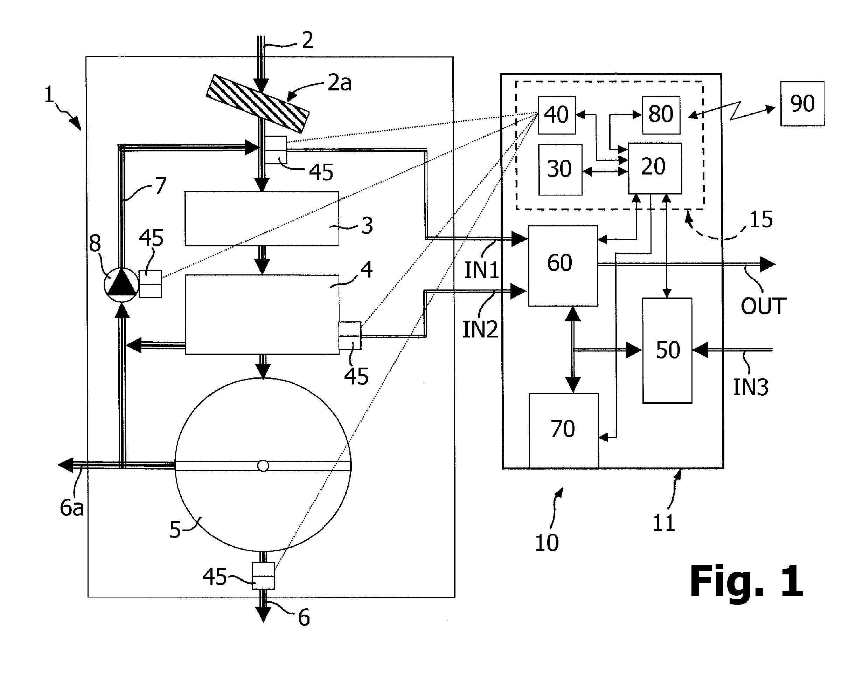

In figure 1, a biological purification plant, which herein is supposed to be a continuous plant working with activated sludge processes, is generally designated by 1, wherein the device according to the invention can be applied.

Figure 1 represents a typical plant engineering approach common to small and also medium activated sludge purifiers, in which one or more similar treatment lines can be obtained in a modular way. The plant 1 has an inlet 2 for the influent or sewage to be treated, connected for example with a sewer system. The influent, after having overcome primary collection and/or lifting and/or sieve processes within the unit indicated by 2a, reaches a first process tank 3, called denitrification tank, wherein the reduction of the nitric nitrogen to gaseous nitrogen takes place. From the tank 3, the sewage is introduced to an oxidation tank 4, where organic carbon and ammonia nitrogen are removed by oxygenation, and therefore to a sedimentation tank 5. The tank 5 performs the separation of the purified effluent, which reaches an exit 6 of the plant, from the exceeding sludge which is partly discharged in 6a and partly recirculated through a conduit 7. Also part of the effluent which passes from the tank 4 to the sedimentation tank 5 is re-circulated in the plant head, through the conduit 7 and a pump 8. Such recirculation mainly serves for optimizing the process in the denitrification tank 3 and therefore the removal of the ammonia nitrogen.

The automatic sampler device subject of the invention, arranged for the monitoring of the bacterial activities and other kinetic parameters typical of the activated sludge plant 1 is indicated as a whole by 10.

As it will result in the following, the device 10 is arranged for the purposes of the automated execution of a plurality of possible analytical experiments, suitable for allowing the monitoring and the control of the biological removal processes. The device 10 is particularly conceived for being used both in continuous-type plants and in sequential SBR-type plants.

The aforesaid analytical experiments are carried out in an automated way through a chemical reactor to which there are operatively associated:
- actuator means, arranged for introducing and/or conditioning in the chemical reactor at least an influent sample and at least a sludge sample coming from the purification plant, as well as a dose of reagent, and
- sensor means, used in the ambit of at least a control action on the aforesaid actuator means.

Through the aforesaid reactor, sensor means and actuator means properly managed by an electronic control system, the device 10 provides for the implementation of the aforesaid analytical experiments in controlled conditions and in a completely automated way.

The sampler device 10 according to the invention is configured for monitoring the kinetics of specific liquid phase biochemical processes through set-point titration methodologies of a DO-stat and pH-stat type, as well as through combinations of such methodologies. The device can be likely arranged also for carrying out set-point titrations of other type, for example Red/Ox-stat (oxide-reduction potential). The device can also be configured for monitoring the kinetics with respirometry techniques.

It has to be remembered that:
- *a **set-point titration*** consists of dosing a chemical reagent suitable for compensating the variation of a chemical-physical parameter which has a monotonous (increasing or decreasing) course, by maintaining such parameter at a constant level; the titration curve represents the volume of dosed reagent over time; the interpretation of these curves, obtained with specific methodologies for any problem, gives back quantitative and qualitative-type information;
- with the titration known as ***pH-Stat,*** an aqueous suspension process which produces or uses acidity is maintained at a pH set-point through the addition of a basic or acidic titrant, respectively;
- with the titration known as ***DO-Stat**,* an aqueous suspension process which uses dissolved oxygen (DO) is maintained at a DO set-point through the addition of a titrant which liberates oxygen (for example through titration with hydrogen peroxide which, in the presence of catalase, a specific and ubiquitary enzyme in biological systems, liberates oxygen).

The device 10 has a control system, indicated as a whole by 15 in figure 1. Such system 15 includes a control unit, represented by the block 20, equipped with a user interface and analog/digital channels, for the connection to sensor means and actuator devices.

The aforesaid user interface preferably includes display means and means for allowing input, by an operator, of commands and data, for the purposes of the configuration of a procedure sequence for the automated execution of one or more analytical experiments. In a preferred embodiment, the above interface is of the type known as *touch-screen.*

Within the control unit 20, a software application is residing, which manages the automated conduction of each analytical experiment, through the control of the sensor means and the actuator means. As mentioned, according to an important characteristic of the invention, the device 10 is arranged for carrying out analytical experiments according to one or more set-point titration methodologies, and particularly according to the pH-stat and DO-stat methodologies, as well as a combination of the same. The device 10 can further be arranged for carrying out analytical experiments with a respirometric and Red/Ox-stat methodology.

To the control unit 20 a computing central unit, represented by the block 30, is operatively associated, which is equipped with the hardware and software resources used for the real-time processing of the acquired signals through the sensor means, their statistical analysis, the application of optimization and patterning methods.

The real-time data processing relates the interpretation of the analytical curves obtained by the experiments above mentioned, carried out according to an analytical methodology amongst those selectable or according to a combination of the same, for the purpose of estimating the values of the bacterial activity, the toxicity of an influent sample, the length of the degradation phase of the different substrates of the plant 1 and the other parameters of interest of the plant. On board of the computing unit 30, a software application of a database type and at least a DSS-type application *(Decision Support System)* are preferably placed. The computing unit 30 is also configured for the purposes of the real-time recording of the acquired signals, the results of their processing as well as the results of each analytical experiment with a possible alarm signaling.

The device 10 includes hydraulic fittings of a per se known type, through which the device itself is connected with one or more hydraulic circuits, for sampling the analytes in one or more points distributed on the plant 1, for the feeding of wash water, for the discharge of the fluids.

In the case exemplified in figure 1, two inlet fittings, diagrammatically represented by the arrows IN1 and IN2 are provided, for the purposes of the connection of the device 10 to pipes for the collection of the fluids present at the exit of the lifting unit 2a (influent) and within the oxidation tank 4 (sludge), respectively. A further inlet fitting for the connection to a water feed network is diagrammatically represented by the arrow IN3. The OUT arrow diagrammatically represents an outlet fitting, for the discharge of the fluids from the device 10.

The control unit 20 is further equipped with electric interface means, represented by the block 40, for the connection, through cables and/or radio-frequency connection, to different kind of sensors and to electric machines or actuator devices, such as pumps and solenoid valves.

Sensor and actuator means associated with the different points of interest of the plant 1 are diagrammatically represented by the blocks indicated by 45. Sensor means relating to the blocks 45 are used for the detection and the control of chemical-physical parameters of the plant 1, whereas actuator means relating to the blocks 45 are those typically provided for the control of the plant (valves, pumps, reagent distributors, such as for example activated carbons), as well as those used for the purposes of collecting from the plant the fluids on which the analytical experiments have to be carried out. Through its interface means, the control unit 20 is further connected with sensor means and actuator means (pumps, solenoid valves, aerators, air-conditioners, etcetera) installed in the device 10 and which relates to:
- an analysis section, represented by the block 50,
- a sampling section, represented by the block 60, and
- a storing and dosage section, represented by the block 70.

To the various sections, pumps and solenoid valves are operatively associated, for the dosage of all liquids used during the experiments, the wash water and the service compressed air, as well as for the discharge of fluids.

The analysis section 50 includes a chemical reactor for carrying out analytical experiments. Such reactor is equipped with at least one electrochemical sensor for the pH measurement, a sensor for measuring the dissolved oxygen (DO), one or more temperature probes. To the mentioned reactor also other sensors can be possibly associated (for example a sensor for measuring the Red/ox reduction-oxide potential, a conductivity sensor, capacitive-type sensors and, more generally, sensors of dielectric and diamagnetic parameters, of luminescence, of electromagnetic spectrum absorbance, of index of refraction, of acoustic reflection and echo).

The chemical reactor further includes an agitation system which can be modulated and a diffuser for insufflating air or other gas.

The dosage of the analytes in the reactor is preferably controlled through level sensors based on electric field measurements, placed on the external walls of the reactor itself and therefore non contacting the internal liquids.

The above mentioned sensor means are of a per se known type.

The sampling section 60 is arranged for the purposes of the taking of the samples to be analyzed which, in the specific case of a purification plant, consist of the influent, or sewage, and the sludge collected in one or more points of the plant, depending on the kind of analyses which one desires to carry out.

The section 70, on the contrary, contains storage tanks for the reagents and titrants, de-ionized water, as well as for detergent and sterilizing substances, used in combination with the water collected from the inlet IN3 for the automated washing of the reactor and the sampling vessels.

Finally, to the control unit 20 communication means are associated, represented by the block 80, for the exchange of signals and data with at least one remote station, represented by the block 90.

The unit 20 concerns the management of all the subsystems of the sampler device 10, and in particular of:
- a system for the controlled and continuous dosage of the titrating solutions; such system is used for introducing, from the section 70 to the section 50, reproducible doses of a titrant required for carrying out pH-stat and DO-stat type-analyses and/or combination thereof (and, if necessary, other set-point titration, for example Red/Ox-stat and/or respirometric titration with a controlled pH);
- a dosage system of pre-determined quantities of chemical reagents solution, from the section 70 to the section 50;
- a dosage system, in the section 50 and the section 60, of the samples to be analyzed;
- a fine thermostatation system in the aforesaid reactor and in the whole analysis section 50;
- an agitation system of the analysis reactor, with a rotation speed control according to selectable profiles, also if varying and programmable during the experiment;
- a user interface, such as a screen of a *touch screen* type, for the manual control of the device 10, the programming and the setting of the experiments, the components calibration, where required, and the local diagnosis (on site);
- a system for the control and the actuation of the external electrical loads (blocks 45 in figure 1) to be used for the sampling of the analytes;
- a system for the control and the actuation of the loads typical of the plant 1 (blocks 45) to be used for the management and the automation of the purification process as a function of the parameters measured and/or computed by the device 10;
- a system for the automatic periodical diagnosis of the device 10;
- a system (block 80 of figure 1) for the management of the connections with the outside of the device 10.

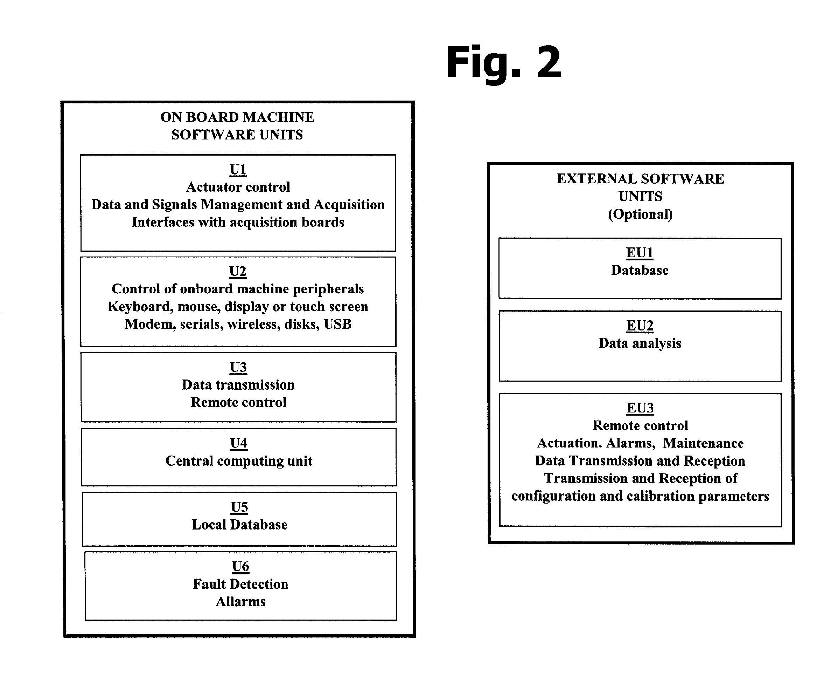

The software implemented within the control system 15, or within the units 20 and 30, preferably includes six program units; three additional program units can be provided for on the external computer 90. It has to be noted that the use of the external computer 90 is merely additional and optional, as the device subject of the invention remains entirely working in the *stand alone* version. The software units are the following (figure 2):

### Unit U1: actuators control and data and signal acquisition

The software belonging to this unit is intended for the control of sensors and actuators existing on the plant 1 and within the device 10. Therefore, pumps, solenoid valves, relays, inverters, encoders and the general electric devices, including alarm and signaling lamps are managed. This software unit further manages data acquisition from the different sensors and their storage in the local database. For this purpose, interface protocols are implemented with sensor and data acquisition devices.

### Unit U2: peripheral control on machine board

In this unit, drivers for controlling the on board peripherals of the device 10 are implemented, such as the user interface (the mentioned *touch screen* or, alternatively, a keyboard, a mouse and a display) and one or more recording units (hard disk).

This software unit also manages a series of USB channels, serial ports RS232/485 of a general and configurable use, as well as one or more amongst a modem connection on a switched line, a RF wireless connection and a GSM, GPRS or UMTS channel (or any other radio-frequency communication system).

### Unit U3: data transmission/remote control

This unit manages data transmission to a remote data logging unit (for example the computer 90) through Internet technologies and protocols, or point-to-point connection with a proprietary protocol. The kinds of network and connections can be of a LAN, WLAN, CAN, MAN, WAN or VPN type, depending on the connectivity requirements of a particular plant.

This unit is further arranged for allowing the remote management of the device 10, for setting controls from a remote position, for the configuration, the observation and the recording of possible operation defects. Such functionality is useful both from the point of view of remote assistance and the normal operation of the device 10 in a remote control mode.

### Unit U4: processing

In this unit there are the implementations of the algorithms for the treatment of the signals, required both for the acquisition from the sensors and probes and for the fine control of the actuator devices.

The implementation of the operating modes (or the analytic experiments) described below is managed through a group of dedicated software modules. Within a module there are proprietary algorithms for the on-line implementation (real-time) of each of the operating modes, as well as the software which handles programmable work cycles constituted by configurable groups obtained by combinations of the operating modes which will be listed below. In particular, the composition of a work cycle has to be considered as a procedural sequence. Each procedural sequence can therefore comprise subsequences containing one of the operating modes which the machine is able to implement.

### Unit U5: local database

This unit manages the local database of the data, as well as the general information on the functioning of the device 10. Such management includes the check of data integrity and the consistency of the information stored.

### Unit U6: alarms

Through this unit the alarms for the safe control of the device 10 and the plant are managed. The generation and the management of the alarms is ensured by a group of modules for the detection of the functioning errors, hardware errors, critical conditions of plant and system.

The following software units are optional and they not necessarily reside on board the sampler device 10, but on one or more personal computers to be considered as system accessories.

### Unit EU1: database

It is the mass database for the storage and the historical analysis of data received by the device 10 from the time of its installation. This database, which can have a more general and complex structure than the one of the unit U5, is provided for the storage and the management of all the available information about the functioning and the story of the plant 1, on the control and maintenance actions carried out by the operators.

### Unit EU2: data analysis software

This software unit is arranged for the display of acquired data, the check and the simulation of experiments, the statistical analysis of results and data. The unit can be connected to the database of Unit EU1, and generate results in a printable and readable format from other computing software, or for the subsequent storage in further databases.

### Unit EU3: remote control (execution, alarms, maintenance, data transmission and reception, transmission and reception of configuration and calibration parameters)

This unit is arranged for allowing the management from a remote user of the device 10 and the actions that have to be carried out on the plant 1. It manages the actuator control, the sending of malfunction alarms to the operator or handler of the plant and it provides for the possibility of remote emergency maintenance interventions; it is further arranged for the purposes of the transmission and reception of locally stored data and the transmission and reception of configuration, functioning and calibration parameters of the device 10.

It has to be noted that the programming specific techniques of the control system, for the purposes of the carrying out of the aforesaid program units, can be of any known type and fall outside the scope of the present invention.

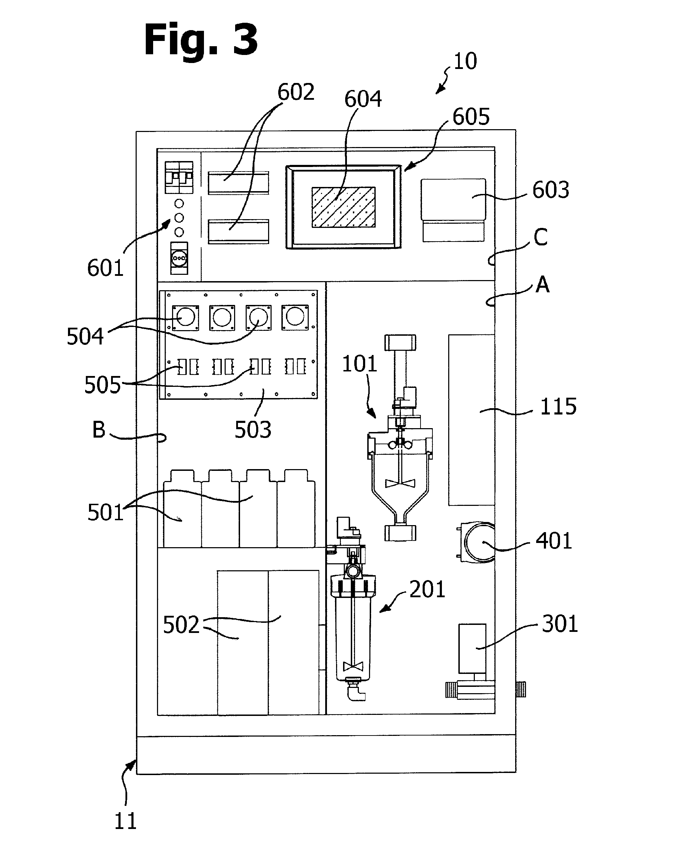

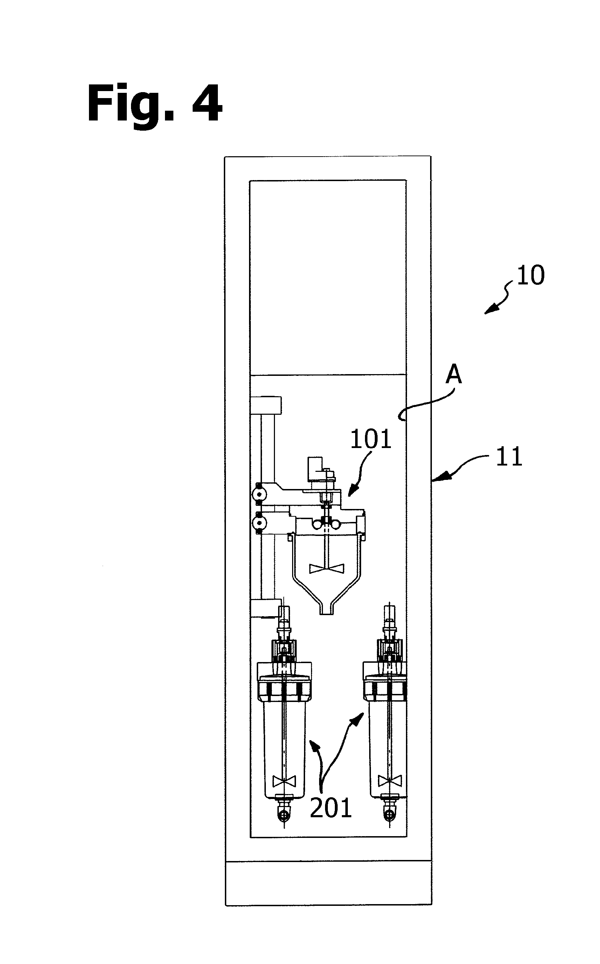

In figures 3 and 4 an example of a sampler device 10 according to the invention is diagrammatically represented. It has to be noted that in such figures hydraulic, electric and pneumatic connections of the different components are not represented for reasons of greater clarity.

The device 10 includes a cabinet 11, within which three distinct compartments, shown by A, B and C, are defined.

The compartment A contains:
- the chemical reactor previously mentioned, indicated as a whole by 101, which forms the main component of the analysis section (block 50 of figure 1) of the device 10;
- two sampling vessels or basins, one for each sampled analyte, indicated by 201, which form the main components of the sampling section (block 60 of figure 1) of the device 10,
- a series of power-driven valves, particularly ball valves, one of which is indicated by 301; in the exemplified case, at least five valves 301 are provided, two of which are for loading fluids to be analyzed within the vessels 201 and three are for the discharge of the reactor 101 and the vessels 201;
- two pumps, preferably of a peristaltic type, one of which is indicated by 401, for the controlled load of the fluids from the vessels 201 to the reactor 101, in pre-determined quantities depending on the working mode or experiment to be carried out.

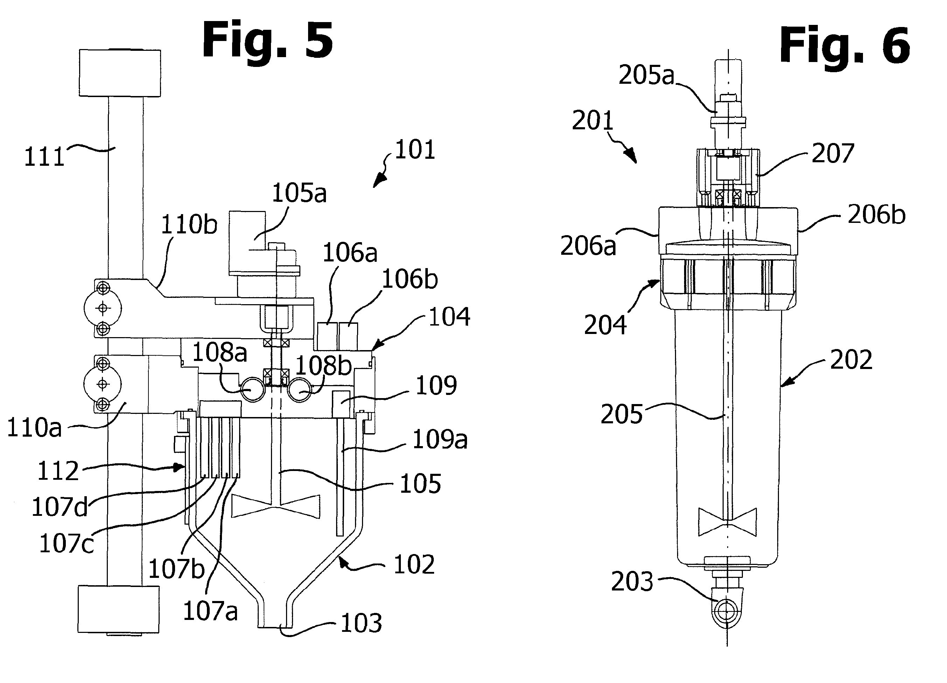

The reactor 101 is used for carrying out the analysis and, as it can be seen in figure 5, presents for this purpose a glass containment body 102 designed according to a cylindrical profile with a conical base, on which bottom a discharge flanged hole 103 is provided, in fluid communication with one of the aforesaid power-driven valves 301, in turn connected with the device discharge (OUT, figure 1).

The upper part of the body 102 is tightly closed with a plastic lid 104, on which there are mounted:
- an agitation bar 105 with a respective motor reducer 105a,
- nozzles for dosing the reagents and a connection with an aerator (for introducing for example air, gaseous oxygen, carbon dioxide, nitrogen) diagrammatically represented in 106a and 106b, respectively,
- sensor means for the programmed analyses, diagrammatically represented; such sensor means include at least an electrochemical sensor 107a, for measuring the pH, a sensor 107b for measuring the dissolved oxygen (DO), at least a temperature probe 107c and, if necessary, one or more sensors 107d of another type, particularly a sensor for measuring the Red/Ox oxide-reduction potential;
- two inlet fittings 108a and 108b for the fluid coming from a respective vessel 201;
- an outlet connection, not visible in figure 5, as an overflow system;
- a cooling module 109.

The body 102 and the lid 104 are supported through respective brackets 110a, 110b movably mounted on a cylindrical rod 111, anchored at the bottom of the cabinet 11. In a possible embodiment, a power-driven system for the lifting of the lid 104 can be provided (for the purposes of maintenance and extraordinary cleaning interventions) and the lowering thereof on the head of the body 102, for the sealed closing during the execution of the analyses.

As mentioned, the control of the levels in the body 102 during the loading of the fluids from the vessels 201 and during the washing is obtained through sensor means 112 based on the electric field measurement, of a per se known type, located outside of the body 102.

Preferably, within the analysis section of the compartment A, a double temperature control is provided for:
- through a classical conditioning device, for maintaining the interior of the compartment A in controlled working conditions, independently of the outer temperature; such device, of a per se known type, is indicated by 115 in figure 3;
- through a fine thermoregulation system of the reactor 101, represented by the module 109, consisting, for example, of a "cold finger" 109a directly inserted in the reactor and working in conjunction with Peltier cells and/or a Vortex tube, for an accurate control of the temperature of the sample under analysis during the experiment.

The motor/reducer block 105 is of electronic control-type and suitably integrates an encoder. The block 105a ensures, through the rod 105, the reproducible and programmable agitation of the suspension under examination and provides the required mechanical energy to the fluid during cleaning at the end of the cycle.

Vessels 201 are used for storing the two fluids to be analyzed in the reactor 101 (sludge and influent), which could be used in different times with respect to their taking, and in the meantime be conditioned through agitation (and likely aeration and/or thermostatation).

The presence of the vessels 201 allows to carry out the taking of the analyte in times differing from those of the analysis by the reactor 101. This is useful particularly in the monitoring of SBR plants, as it results possible to collect a given analyte at a predetermined time of the plant phase and to analyze it in a second time, when the reactor 101 is available for a new analysis.

One of the sampling vessels 201 is visible in figure 6.

The vessel 201 has a substantially cylindrical transparent body 202, for example made of plastic, on whose bottom a fitting 203 is provided, connected - with interposition of one of the valves 301 - to a corresponding inlet IN1, IN2 for the sampling of the analyte (IN1 or IN2 in figure 1). Through two T-shaped fittings interposed between the connection 203 and the inlet valve IN1, IN2, the fluid is alternatively directed to the discharge OUT of the device 10 (figure 1) or to a respective inlet 108a or 108b of the reactor 101. In case of emptying of the content of the vessel 201, another one of the valves 301 is opened, whose exit is connected to the discharge OUT; vice versa, in case of transfer of the content of the vessel 201 to the reactor 101, the peristaltic pump 401, connected with the other T-shaped fitting in series with the first one, will be operated, the exit of such pump being connected to one of the inlets 108a, 108b of the reactor 101.

The upper part of the body 202 is seal-closed with a lid 204 made of plastic, on which an agitation rod 205 with a respective motor reducer 205a is mounted. The lid 204 has an inlet fitting 206a for the wash water and an outlet fitting 20b as an overflow system. Reference 207 designates a support bracket of the vessel 201, anchored with a wall of the cabinet 11.

Vessels 201 can be likely arranged, besides pre-treatment of the analytes through agitation, also for their thermal conditioning, for the insufflation of air, oxygen, carbon dioxide or an inert gas. In such a case, to the lid 204, a thermostatation system and at least an inlet of the used aeriform medium are associated.

The compartment B, corresponding with the storage and dosage section, contains storage tanks, some of which are indicated by 501, for each reagent, detergent and sterilizing agent, and a storage tank 502 for each titrant (for example four reagents, one detergent, one sterilizing agent and two titrants).

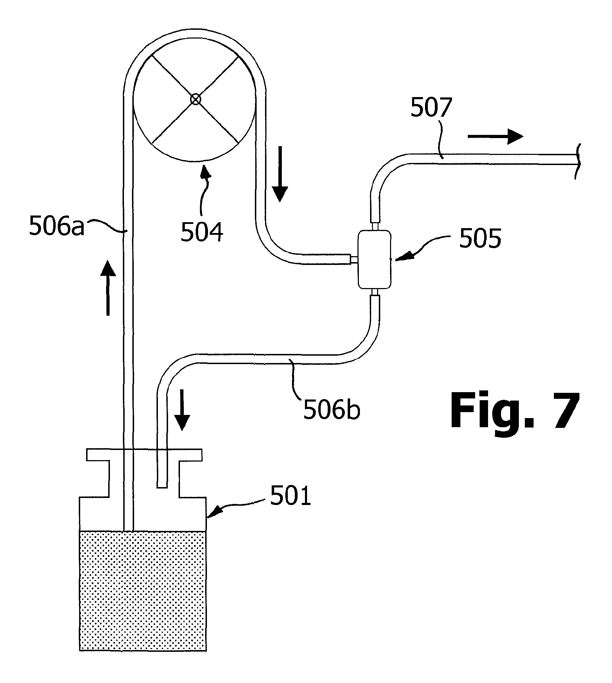

The compartment B further includes a support 503 of the controllable components of the hydraulic system for the dosage of the reagents, titrants, detergent and sterilizing agent. In particular, a series of peristaltic pumps 504 and a series of three-way solenoid valves 505 are provided. In the example, four two-channel peristaltic pumps and eight solenoid valves are foreseen, for a total of eight dosage lines:
- a peristaltic pump and a solenoid valve for each reagent and titrant to be dosed in the reactor 101;
- a peristaltic pump and a solenoid valve for the dosage of the detergent;
- a peristaltic pump and a solenoid valve for the dosage of the sterilizing agent.
   The dosing diagram for each of the reagent or titrant solutions is shown in figure 7. The liquid contained within the tank indicated by 501, such as a can, is drawn off by the respective peristaltic pump 504 and sent to the three-way solenoid valve 505. In the preferred embodiment, the valves 505 are total separation solenoid micro-valves, controlled by the module 40 with a configurable duty cycle. When the solenoid is resting, the fluid is circulated in the storage tank 501 through the conduits 506a-506b; when, on the contrary, the solenoid is excited, the flow is deviated in the conduit 507, which leads to the reactor 101. The dosage is carried out by discrete doses, starting a rapid duty cycle (of the order of seconds); the dosed quantity is proportional to the number of opening and closing cycles of the solenoid micro-valve 505 and to the flow rate imparted to the liquid by the peristaltic pump 504. The flow rate is determined through a periodical calibration procedure on each channel. The described arrangement allows to obtain a precise and reliable dosage.
   The compartment C, relating to the control system 15 of the device, contains
- at least a switch (ON/OFF) and control lamps, indicated as whole by 601;
- at least a communication module; in the example shown, two modules of a GSM type indicated by 602 are provided (which form the block 80 of figure 1);
- a data interface, acquisition and transduction module of the sensors provided for the reactor 101 (and likely similar sensors foreseen in the vessels 201), indicated by 603;
- a user interface with a touch screen-type display, indicated by 604;
- a central processing unit, indicated by 605.

The central unit 605 is equipped with a microprocessor motherboard, preferably having hard disk and/or other mass storage support, possibly of a removable type. The unit 605, through the respective computing unit (block 30 of figure 1) is arranged for the treatment of the collected data through the sensors existing in the analysis compartment A and on the plant 1, and for their recording on the database type application above mentioned. The unit 605 is further configured for controlling all the electrical components of the device 10, including herein the user interface 604, and of the plant 1, through modules not shown in the figure (which form the block 40 of figure 1).

Processed and recorded data are made available for the actuation of likely loads or controls on the purification plant 1, or for alarm signaling. Data are further made available to a group of applications, structured according to the architecture known as client/server, so as to carry out, through the communication module 602, a remote access mode to the sampler device 10, through the implementation of one or more protocols amongst those commonly used in the Internet technology and such that these data can be transmitted, where foreseen, to an application of a remote database type and made available to authorized users.

On the central unit 605 all the control algorithms suitable for carrying out the analytical experiments with the modes described below are implemented. Each analytical experiment is characterized both by a proper configuration of the acquired data, in number and type, and by a specific preparation procedure of the sample in the reactor 101, as well as a particular sampling method.

Data are acquired and processed in real time and allow an instantaneous control of control reactions and actions on the plant 1, where foreseen. By means of the implementation of statistical patterns and with the use of the computed parameters, the device 1 can in fact carry out processing suitable for promoting the process optimization and the automatic control of the plant 1.

Also the titration curves, generated by the graphical representation of the quantities of the reagent/titrant dosed in function of time, are analyzed in real time and allow to discover the characteristic points relating to determined biochemical events (for example the end of the nitrification or denitrification phase of a sewage in a SBR-type reactor).

Through the information collected within the local and/or remote database, the device 1 is able to implement auto-tuning procedures (to be intended, in particular, as auto-calibration of the device 10 based on parameters measured or extrapolated by the titration curves).

As mentioned, thanks to the mentioned control unit and computing unit, the control system of the device 10 is able to implement a procedural sequence of processing and actions, properly developed as a function of the kind of control or measure to be executed in a particular process. Such a sequence is configurable as a timed and automated composition of subsequences, which carry out a series of operating modes. Each operating mode is automatically carried out through activation of the actuator devices (valves, pumps, aerators, stirrers, etcetera), which provide for the introduction of the influent and the sludge to be analyzed to the sampling vessels 201 and the reactor 101, and through the sensor devices (pH, dissolved oxygen, etcetera) which acquire the chemical-physical signals.

The pH-stat and DO-stat titrations can be applied according to multiple variants, which allow to determine different kinetic parameters of a same biochemical reaction or characterize biochemical reactions of a different kind. Generally, each of the possible experiment which can be executed provides for the sequence of the four following steps:
i) the biological process under examination is reproduced in the chemical reactor, so as to comprise the same substrates and/or the same biomasses involved in said process. In particular, herein the applications of interest involve the use of biomasses coming from the biological purification plant 1, such as heterotroph and/or autotroph bacterial populations, which oxidize organic compounds of the carbon and/or ammonia nitrogen to nitrous nitrogen and/or the nitrous nitrogen to nitric nitrogen and reduce the nitric nitrogen to nitrous nitrogen;
ii) a pH-stat titration or a DO-stat titration, or contemporaneously a pH-stat titration and a DO-stat titration, are carried out on the whole biomass/substrate suspension system;
iii) dosage rates of titrants used during the development of the pH-stat, DO-stat or pH-DO-Stat titrations are acquired;
iv) titration curves are constructed, with a graphical representation of the volumes of each titrant as a function of the time. On each curve, the time (T0) is detected in which the signals (pH, DO) have reached the assigned set-point value, and other characteristics points concerning the kind of developed analysis are likely detected. Data obtained from the curves are processed by the processing unit with proper stoichiometric formulas, depending on the investigated parameter.

An exemplifying and not exhaustive list of possible automatically operating modes performed by the sampler 10 is the following.

### Mode 1: evaluation of the acute toxicity of the influent towards the autotroph biomasses responsible of the removal process of the ammonia nitrogen

Through a pH-DO-Stat type-experiment, this mode allows to evaluate the presence of inhibiting/toxic substances with acute effects on the autotroph biomasses, both ammonia- and nitrite-oxidants. The activities estimated in the last test are compared with historical data of preceding tests. In case of a higher toxicity with respect to a predefined threshold, an alarm signal and, if necessary, a feedback control are produced.

### Mode 2: estimation of the nitrificable nitrogen on the influent

Through a pH-DO-Stat type-experiment, this mode allows to rapidly obtain an evaluation of the content of nitrificable nitrogen existing in the entering sewage. Such information is used, for example, for facing nitrogenous overload situations through an increase of the aeration flow rate in the plant; vice versa, in case of an ascertained sub-load, data can be used for implementing energy saving strategies always acting on the aeration devices.

### Mode 3: estimation of maximum nitrifying activity

Through a pH-DO-Stat type-experiment, this mode allows to estimate the maximum specific activities of the oxidizing ammonium and oxidizing nitrite biomasses of the sludge existing in the reactor 4, 5 of interest of the purification plant 1. By recording the collected daily data, it is possible to obtain pre-alarms on risk situations concerning the healthy state of the autotroph biomasses and foresee malfunction events.

### Mode 4: determination of the denitrifying capacity of the influent

Through a pH-DO-Stat type-experiment, this mode allows a rapid check of the specific load of nitric nitrogen (in gN per liter of sewage) which can be reduced from the entering sewage. Such indication can be advantageously exploited by the control system of the device, in a control logic of a "feed-forward" type, in order to control the likely dosage in the plant 1 of external carbon sources, in case of lack of carbon of the influent, required for respecting the nitric nitrogen limit within the effluent.

### Mode 5: estimation of the nitrificable nitrogen on the effluent

Through a pH-DO-Stat type-experiment, this mode allows to rapidly obtain an evaluation of the nitrificable nitrogen content existing in the purified fluid. This allows to indirectly check if the purifying treatment allows or not to respect the discharge limits of the ammonia nitrogen.

### Mode 6: estimation of the rapidly biodegradable COD value of the sullage

Through a pH-DO-Stat type-experiment, this mode allows to measure the concentration of the biodegradable fraction of the organic substance contained within the influent sewage, providing the opportunity of detecting overloads, if any, manageable with proper feedbacks.

### Mode 7: estimation of nitrates in the final effluent

Through a pH-DO-Stat type-experiment, this mode allows to estimate the residual concentration of nitrates in the effluent. Such an indication can advantageously be used for checking likely excesses of nitrogen oxides (nitrites and nitrates) in the final effluent and carry out corrective measures (for example: dosage of organic carbon rapidly biodegradable within the denitrification tank).

### Mode 8: determination aerobic phase end (only SBR-ASP)

Through a pH-DO-Stat type-experiment, this mode, which can be used only in case of a SBR-type plant, allows a rapid check concerning the completion of the oxidation process of the ammonia nitrogen during the aerobic phase. Such indication can be advantageously exploited for the optimization of the aeration times of the plant.

### Mode 9: nitrates estimation at the beginning and/or end of the anoxic phase (only SBR-ASP)

Through a pH-Stat type-experiment, this mode, which can be used only in case of a SBR-type plant, allows to estimate the residual concentration of nitrates at the end of the oxidation step or at the end of the anoxic step. The data can advantageously be used in order to foresee the length of the denitrification process of the following anoxic phase and determine likely accumulations of nitrogen oxides (nitrites and nitrates).

### Mode 10: nitrates estimation in the mixture in the anoxic and/or aerobic compartment (only CF-ASP)

Through a pH-Stat type-experiment, this mode, which can be used only in case of a continuous plant, allows to estimate the concentration of nitrates in the anoxic and/or aerobic compartment. The data can advantageously be used for optimizing recirculation of the aerated mixture and the functioning of the blowers for the purposes of the optimization of the nitrogen removal process.

### Mode 11: estimation of the carbonate alkalinity and the concentration of volatile fatty acids (VFA)

This mode allows to determine parameters of a fundamental importance in the management of anaerobic digestors, such as the VFA concentration and the carbonate alkalinity. Estimated data are used for the purpose of preventing VFA accumulation phenomena which can easily and rapidly cause the inhibition of the methanogenesis process and therefore compromise the effectiveness of the digestive process.

From what above described, it can be inferred how the sampler device 10 according to invention is suitable for monitoring the kinetics of biochemical processes in a liquid phase through pH-Stat methodologies and pH-Stat and DO-Stat combinations.

The described sampler device allows the monitoring of kinetic parameters in different compartments of the plant 1, playing a timely monitoring and control, also by a remote location. This is particularly useful in plants of small and medium dimensions, wherein the continuous presence of operators in loco in not provided for.

The described automatic sampler conjugates the requirements of inexpensiveness, easiness of use, integration with the plant system with those of reliability, reproducibility, action spectrum, fault detection being typical of complex and expensive real-time control systems. These characteristics make possible to interface the device also on smaller plant, which otherwise, for their control, would not justify the use of too expensive systems for their scale economies.

The predisposition of being integrated to a central supervisory system through telecommunication instruments allows to carry out a central supervisory system of more plants and/or more treatment lines. In the particular situation in which a company has to manage various medium-small plants distributed on the territory, the use of the sampler according to the invention constitutes a solution perfectly integrated with the scale economies of these plants, which can also be integrated on obsolete structures with not many predispositions and able to ensure the supervisory and intervention performances required in a modem plant by the novel regulations.

The device finds use in water treatment plants, in particular in the biological removal processes of nutrients, both in continuous plant and in batch plants. Amongst these latter, SBR-type plants find an optimal union with the device according to the invention as, with a single measurement and control instrument of compact dimensions, it is possible to ensure the main requirements of this kind of plants. In particular, the sampler device 10 can play, on a SBR plant, the following actions:
- measurement of the kinetic parameters of the involved biomasses;
- monitoring on the influents for preventing inhibition phenomena;
- monitoring of the polluting load (carbon and nitrogen) entering and exiting the plant;
- adaptive adjustment of the temporal length of the typical sequences (load, anoxic phase, oxidative phase, settling or discharge), as a function of the entering polluting load and the current chemical-physical conditions (working temperature, bacterial activity, presence of inhibiting compounds, etcetera);
- maximizing the working flow rates for the purification, by minimizing the energy consumption;
- effectively preventing malfunction events due to damaging of the biomass existing in the sludges, through the timely detection of the risky situations.

By installing the sampler device 10 on a SBR-type purification plant it is therefore obtained a system able to automatically manage all the biological removal process, without the help of further equipments. The device 10 can detect the succession of the biological phases of this kind of process and can control in an intelligent way the actuators of the plant, thus being able to manage the process phases without using the classical timing systems. This involves a definite improvement of the process, with results on the final quality of the effluent and an economic saving due to the greater load of treated influent in the same planimetric conditions of the plant and to the safe in energy consumption resulting from the optimization of the aeration phase. Furthermore, the malfunction risks and the restoring costs due to a not timely intervention on the process are reduced.

Analogously to what above mentioned, the particular functionalities of the automatic sampler device according to the invention can be profitably exploited also in other kinds of waste water treatment plant, such as for example adhered biomass type-plants and MBR *(Membrane Biological Reactor)-type* plants.

## Claims

1. Automatic sampler device for an activated sludge waste water treatment plant, including:
- an electronic control system (15),
- fitting means (IN1, IN2) for the hydraulic connection of the device to a treatment or purification plant (1), for the purposes of the controlled taking therefrom of an influent sample, or a first analyte, and a sludge sample, or a second analyte,
- storage means (501, 502) for one or more reagents and/or titrants,
- a chemical reactor (101), in fluid communication with said fitting means (IN1, IN2) and with said storage means (501, 502),
- actuator means (105, 106, 109, 301, 401), adapted to be controlled by said control means (15) for the purpose of adducing and/or conditioning within said chemical reactor (101) at least a dose of a said analyte and at least a dose of reagent or titrant,
- sensor means (107, 112), operatively associated with said chemical reactor, for the detection of quantities relating to the content of the reactor, said sensor means being particularly used by the control system (15) in the ambit of at least a control action on said actuator means (105, 106, 109, 301, 401),
wherein said control system (15) is arranged for implementing in said chemical reactor (101), through said sensor means and said actuator means, one or more analytical experiments in an automated way, using at least one set-point titration methodology selected from pH-Stat titration and DO-Stat titration.

2. Device according to claim 1, wherein said control system (15) is arranged for implementing in said chemical reactor (101), through said sensor means and said actuator means, one or more analytical experiments in an automated way, using at the same time the pH-Stat and DO-Stat titration methodologies.

3. Device according to claim 1 or 2, wherein said sensor means (107, 112) comprise at least a pH sensor (107a), a dissolved oxygen concentration sensor (107b), a temperature sensor (107c) and possibly a Red/Ox sensor.

4. Device according to claim 1 or 2, wherein said actuator means comprise at least a dosage system (503-507) for adducing in said chemical reactor (101), in a batch way, reproducible amounts of an acidic reagent and/or a basic reagent and/or an oxidizing reagent and/or a reagent which liberates oxygen.

5. Device according to claim 1 or 2, wherein said sensor means (107, 112) comprise at least a meter (112) of the level the content of said chemical reactor (101), said level meter particularly operating on the basis of electric field measurements.

6. Device according to claim 1 or 2, wherein said actuator means comprise at least a dosage system (106b) for admitting in said chemical reactor (101) an aeriform medium, such as air, gaseous oxygen, carbon dioxide, nitrogen.

7. Device according to at least one of the preceding claims, wherein said actuator means comprise at least an agitation system (105, 105a) of the content of said chemical reactor (101).

8. Device according to at least one of the preceding claims, wherein said actuator means comprise at least a thermoregulation system of the content of said chemical reactor (101).

9. Device according to at least one of the preceding claims, further comprising at least a sampling vessel (201), operatively interposed between one said hydraulic fitting means (IN1, IN2) and said chemical reactor (101), the sampling vessel being designed for containing a respective analyte sample before the execution of a said analytical experiment within said chemical reactor (101).

10. Device according to claim 9, wherein each sampling vessel (201) has a fitting (203) in a fluid communication with a respective hydraulic fitting means (IN1, IN2) which can be selectively connected to a discharge channel (OUT) of the device and to a connection conduit, in particular served by a pump device (401), with an inlet (108a, 108b) of said chemical reactor (101).

11. Device according to claim 9 or 10, wherein each sampling vessel (201) is associated with a respective agitation system (205, 205a) of the content of the vessel.

12. Device according to claim 10, wherein said actuator means (105, 106, 109, 301, 401) comprise a pump (401), in particular a peristaltic pump, operating along said connection conduit for the load of said chemical reactor (101).

13. Device according to claim 9, wherein said chemical reactor (101) and said sampling vessels (201) are mounted in a same compartment (A) of a cabinet (11) of the device, said compartment being equipped with an environmental air-conditioning system.

14. Device according to at least one of the preceding claims, further including storage means (501) for at least one of a detergent substance and a sterilizing substance.

15. Device according to at least one of the preceding claims, wherein each sampling vessel (201) has an inlet (206b) for a detergent and/or a sterilizing fluid.

16. Device according to at least one of the preceding claims, wherein said control system (15) comprises a user interface (604), for the input of commands and data by an operator and the displaying of information.

17. Device according to at least one of the preceding claims, wherein said control system (15) further comprises means (602) for signals and data exchange with a remote control unit (90).

18. Device according to at least one of the preceding claims, wherein in said control system (15) there resides one or more software applications comprising one or more of the following:
- an application for the automated conduction of said analytical experiments through the control of said sensor means and actuator means;
- an application for the processing of signals acquired through said sensor means, said processing particularly concerning acquisition curves of a said analytical experiment for the purpose of determining the value of kinetic and/or stoichiometric quantities typical of biological and/or chemical reactions of the treatment or purification plant;
- an application for the statistical processing of data;
- an application for a real-time recording and classification of the analytical results, with a likely alarm signaling;
- an application for the remote connection and/or the remote control of the sampler device;
- a database type-application;
- a DSS (decision support system) type-application.

19. Device according to at least one of the preceding claims, wherein said analytical experiment is aimed to at least one of the following estimations and/or determinations:
- estimation of the acute toxicity of said influent sample and/or detection of the presence of inhibiting/toxic substances in said influent sample;
- estimation of the nitrificable nitrogen quantity of said influent sample;
- estimation of the maximum nitrifying activity of said sludge sample;
- estimation of the denitrifying capacity of said influent sample;
- estimation of the nitrificable nitrogen in the effluent of the treatment or purification plant;
- estimation of the concentration of the rapidly biodegradable COD fraction;
- estimation of the residual concentration of nitrates within the effluent of the treatment or purification plant;
- determination of the completion of the oxidation reaction of ammonia and nitrous nitrogen in SBR-type reactors;
- estimation of the residual concentration of nitrates at the end of the oxidation phase or at the end of the anoxic phase in SBR-type reactors;
- determination of the concentration of nitrates in the anoxic compartment and/or the aerobic compartment of a continuous-type treatment or purification plant;
- estimation of the carbonate alkalinity and the concentration of volatile fatty acids.
The whole substantially as described and shown, and for the stated purposes.
